# EUROPEAN PATENT SPECIFICATION

(11) **EP 3 065 558 B1**
(45) Date of publication and mention of the grant of the patent: **24.04.2019**
(21) Application number: 14802938.2
(22) Date of filing: 10.11.2014
(51) Int. Cl.: A23K 10/10, A23K 10/40, A23K 50/00, A61P 1/04

(54) **FEEDSTUFF FOR EQUIDS**
FUTTERMITTEL FÜR EINHUFERN
ALIMENT POUR ÉQUIDÉS

(30) Priority: 08.11.2013 GB 201319796
(43) Date of publication of application: 14.09.2016
(73) Proprietor: Johnston-Murphy, Sandra, Market Rasen, Lincolnshire LN7 6NL (GB)
(72) Inventor: Johnston-Murphy, Sandra, Market Rasen, Lincolnshire LN7 6NL (GB)
(74) Representative: Loven, Keith James
(86) International application number: PCT/GB2014/053332
(87) International publication number: WO 2015/067967

(56) References cited:
- WO-A1-2014/035470
- WO-A2-2008/041835
- AU-B2- 2011 211 461
- US-A1- 2008 095 881
- US-A1- 2009 181 114
- US-A1- 2010 291 050
- US-B1- 6 827 965
- PEIRETTI P G ET AL: "Fatty acid and nutritive quality of chia (Salvia hispanica L.) seeds and plant during growth", ANIMAL FEED SCIENCE AND TECHNOLOGY, ELSEVIER, AMSTERDAM, NL, vol. 148, no. 2-4, 16 January 2009 (2009-01-16), pages 267-275, XP025860086, ISSN: 0377-8401, DOI: 10.1016/J.ANIFEEDSCI.2008.04.006 [retrieved on 2008-05-27]
- "Tri-Omega supplement ED - Squires Edward", JOURNAL OF EQUINE VETERINARY SCIENCE, JONES, WILDOMAR, CA, US, vol. 18, no. 6, 1 June 1998 (1998-06-01), page 367, XP022330420, ISSN: 0737-0806, DOI: 10.1016/S0737-0806(98)80560-1

## Description

### Field of the Invention

The present invention relates to an animal foodstuff. The scope of the present invention is defined by the claims.

### Background to the Invention

The small intestine is the initial site for water absorption and therefore free water molecules will be taken easily through the semi permeable membrane onto the interstitial fluid to service such systems as thermoregulation, digestion, excretion, mucous membrane and saliva production. This system is efficient at taking all available water and utilising it as quickly as possible to service these systems whilst idle (at maintenance).

However, the demands on this system are stretched when the animal is asked to perform over and above maintenance. Because there is no storage in the interstitial fluid, when exertion or increased fluid requirements , i.e. exercise, heat stress or dry feed stuff, extra fluid is needed to replace fluid lost in sweat or saliva production. If there is very little fluid available in the hindgut, then this fluid is drawn from the blood plasma, which ultimately will cause a fluid deficit and dehydration will ensue.

A normal wet feed will carry a small amount of water through to the hindgut attached to soluble fibre, for instance alfalfa, beet pulp etc., however not enough water is carried thorough as saturation occurs relatively quickly because there is not enough soluble fibre available for the free water to bind with.

Across the world animals such as horses live, work and travel in ambient temperatures well in excess of room temperature. Without an intake of at least 30 litres of water a day, then a normal 500kg horse will be in danger of dehydration. Further issues arise when the horses are required to perform strenuous exercise, as this further depletes body fluid and other nutrients. Dehydration in racehorses and horses performing endurance events is well documented. The current solution is to offer the horses water during and after exercise. However it is not possible to force a horse to drink of its own accord, and invariably the horse takes on too little water to counteract the problem. In severe cases the horse can be fed a bolus of fluids through a feeding tube directly into the stomach, or even put on an intravenous drip as a means of rehydration. It is clear therefore that none of these solutions are satisfactory and the saying "you can lead a horse to water, but you can't make it drink" is almost certainly true. Unfortunately, in many cases there are detrimental effects to the horse for lack of hydration, in particular a reduction in performance, and sudden death.

### What makes this formulation appeal to Equids?

Equids have been around for Millennia, roaming the moorlands, woodlands and plains of the world, the ancient horse was not constrained to modern paddocks or confined to stables where forage is limited and water is provided by bucket, water drinkers or troughs. The animals roamed freely using their natural instinct to forage to gain sustenance. The digestive system of the animal evolved over millions of years to process large amounts of fibrous plant material and relatively small amounts of carbohydrate and starchy materials and therefore derived all the essential nutrients, vitamins and minerals necessary to sustain life and to thrive without exertion or stress (maintenance). This included the most important nutrient - water. The water too was derived by forage from water contained within the plant material and from morning dew, snow, and raindrops that settled on the forage.

The forage was broken down by the molars which released free water molecules from the plant material absorbed through the semi permeable membrane of the small intestine, (the initial site of water absorption) and into the body fluids to be utilised to balance the body fluid (fluid homeostasis). This process replaced the natural water losses, such as that lost in saliva and mucous membrane, digestion, excretion and thermoregulation. This efficient system of fluid restoration was very important as the animal may have had to maintain its systems for long hours before it was able to find a source of pure water to replace its natural losses whilst roaming without any particular stress. However if the horse had to flee from a predator or endure increased temperature, then the hydration status of the animal would be compromised, therefore the water within its digestive system would be utilised to provide the necessary fluid to allow for escape or to prevent collapse, however if the digestive system was unable to give up any water, then the body would resort to utilising water from the body fluids causing a fluid deficit (dehydration) which would make the animal weak and certain death would ensue.

Fluid balance is therefore very important which is why it is linked to the forage instinct, however to be able to replenish the larger amounts of water required to be replaced after or during stress such as heat or exertion over and above maintenance, the animals have evolved a system or mechanism that allows this rapid restoration of intracellular and extracellular fluid deficits. Neural signals received by osmotic signals on the lamina terminalis are integrated in the brain, with afferent information relayed from mechanoreceptors via the hypothalamus to generate thirst. These osmotic and mechanoreceptors are components of the regulatory mechanism that provide the animal with an urge to drink. However this is a regulatory response rather than an instinct.

In the case of osmotic signals, a loss of sodium chloride (NaCI) the major solute of extracellular fluid together with water (such as in thermoregulation, i.e. sweating or diarrhoea) results in proportionately more extracellular fluid being depleted than if water alone is lost. Also with ingestion of dry matter which extracts water from the digestive system and the increase in saliva production for lubrication causes a deficit in fluid balance which switches in the thirst mechanism.

If the horses has to increase its exertion or experiences heat stress over and above maintenance and there is not enough fluid within the digestive system to service the body fluid homeostasis, the necessary fluid needed is therefore taken from the blood plasma. Simply explained, the blood plasma volume becomes more concentrated causing it to become more viscous, this makes the heart rate increase in an attempt to pump the thickened blood around the body, which is exacerbated if a splenic release of red blood cells occur. This increase in heart rate which increases the respiratory rate and so causes an increase in CO₂ production, which can lead to respiratory acidosis and hyperventilation.

The lack of oxygen getting to the muscles causes lactic acid to build up resulting in fatigue. This process of events will slow the animal down and leave it vulnerable to capture by a predator and death may quickly ensue in the wild. However domesticated animals asked to perform strenuous exercise or to work in temperatures over and above thermal neutral zones will experience these same detrimental effects and therefore are susceptible to loss of performance, fatigue and in some cases sudden death.

The formulation comprising of a water component, a fibrous component, a carbohydrate component and an edible gel component suspended within an aqueous solution which is highly palatable (according to a nationwide palatability trial carried out August 2013 using 100 horses, which showed 98% of horses ingested the formulation immediately). It appeals to the horse's natural instinct to forage which dispels the myth that 'you can lead a horse to water but you can't make it drink'.

Because the formulation cannot be masticated due to its small particle size (< 2mm) the forage therefore has to be drunk. This allows control of the amount of water administered to the horse using this method of delivery. The formulation rate of absorption allows water to be retained within the bolus for longer therefore delivering hyperhydration to the hindgut making fluid available for fluid homeostasis in times of heat or exertion stress.

Below is provided a description of the problems associated with hydrating horses.

### (1) Horses working or performing in temperatures above 20°C and operating above their thermoneutral zone.

Many equines, including donkeys, mules and asses, live and work in extreme temperatures. With the exception of inspired oxygen, a deficiency of water produces death more rapidly than a deficiency of any other substance. Across the globe many equines work in high ambient temperatures and operate above their thermal neutral zones. This can increase water intake by as much as 300 - 400% caused by acute and chronic heat loads and exercise and in these extreme temperatures these animals have to contend with the effects of dehydration on a regular basis. Donkeys have adapted a system to store water within the gastrointestinal tract much more efficiently than horses apart from specific types of Arabian breeds, as well as the ability to converse blood volume which would not be possible in many other species of mammal. Signs of dehydration may not be apparent until a 6% loss of body weight occurs. However, it is thought that the physical performance of the horse is impaired between 3-5%. Also, dehydration causes plasma, electrolyte, urea and other body fluids to become concentrated and blood volume to decrease reducing blood flow to the muscles resulting in fatigue. This type of fatigue requires several days for complete repletion of water and electrolytes for recovery.

### 2) Transportation of equines over long distances and in hot climates above 30°C

This includes the transportation of horses over long distances, typically more than 5 hours, whilst confined in vehicles, aircraft or on board a ship in hot climates or ambient temperatures above 30°C. Many horses often refuse to eat or drink while traveling and may lose upwards of 5% of their body weight, enough to become clinically dehydrated, especially if these animals are intended for slaughter as they may not have been in good condition before the journey and the time scale of previous feed and water intake may be unknown. Many of these horses display colic symptoms due to dehydration which occurs when horses lose body water, in the form of sweat or losses attributed to ingestion of hay that is being feed on the journey causing their digestive tracts to become dehydrated which carries a high risk of impaction colic. Upon rehydration, body fluids concentrations return to normal relatively quickly, however rehydration also has its dangers as severely dehydrated horses can suffer colic symptoms due to rapid replacement of the gastrointestinal fluid pool and possible water toxicity.

### 3) Performance and endurance horses

For the majority of performance horses, increased amounts of energy are required to compensate for the increased levels of exercise. These high energy demands are usually met be increased intake of grain and a decrease in forage. However an increase in grain supplementation causes an increase in acid production which increases the risk of gastric ulcers, founder and exertional myopathy. Therefore supplementing high grain diets with dietary fats increases the energy density of the diet allowing the horse to consume more dietary energy without a proportionate increase in feed intake. However decreasing forage intake decreases water, electrolytes and energy supplying nutrients in the gastrointestinal tract; these are beneficial for endurance. The increase in dietary fat decreases the amount of energy used for heat production and increases the energy available for physical activity and glycogen storage and utilisation. As a result of fat supplementation it has been shown to enhance both anaerobic and aerobic performance activities and delay the onset of fatigue. However a high fat diet decreases heat production by as much as 14%.

### Colic

Colic is a term used to describe a symptom of abdominal pain, which in horses is usually caused by problems in the gastrointestinal tract.

There are over 70 different types of intestinal problems that cause colic symptoms, which range from mild to severe (life-threatening) in nature. Among domesticated horses, colic is the leading cause of premature death. The incidence of colic in the general horse population has been estimated between 4% and 10% over the course of their lifetime. Clinical signs of colic generally require treatment by a veterinarian.

Colic can be divided broadly into several categories:
a. Excessive gas accumulation in the intestine (gas colic).
b. Simple obstruction (impaction colic).
c. Strangulating obstruction.
d. Non-strangulating infarction.
e. Inflammation of the gastrointestinal tract (enteritis, colitis) or the peritoneum (peritonitis).
f. Ulceration of the gastrointestinal mucosa.

These categories can be further differentiated based on location of the lesion and underlying cause.

### Gas and Spasmodic colic

Gas colic, also known as tympanic colic, is the result of gas buildup within the horse's digestive tract due to excessive fermentation within the intestines or a decreased ability to move gas through it. It is usually the result of a change in diet, but can also occur due to low dietary roughage levels, parasites (22% of spasmodic colics are associated with tapeworms), and anthelminthic administration. This gas buildup causes distention and increases pressure in the intestines, causing pain. Additionally, it usually causes an increase in peristaltic waves, which can lead to painful spasms of the intestine, producing subsequent spasmodic colic. The clinical signs of these forms of colic are generally mild, transient and respond well to spasmolytic medications, such as buscopan, and analgesics. Gas colics usually self-correct, but there is the risk of subsequent torsion (volvulus) or displacement of the bowel due to gas distention, which causes this affected piece of bowel to rise upward in the abdomen.

Abdominal distention may occasionally be seen in adult horses in the flank region, if the cecum or large colon is affected. Foals, however, may show signs of gas within the small intestines with severe abdominal distention.

### Simple obstruction (Pelvic flexure impaction)

This is caused by an impaction of food material (water, grass, hay, grain) at a part of the large bowel known as the pelvic flexure of the left colon where the intestine takes a 180 degree turn and narrows. Impaction generally responds well to medical treatment, usually requiring a few days of fluids and laxatives such as mineral oil, but more severe cases may not recover without surgery. If left untreated, severe impaction colic can be fatal. The most common cause is when the horse is on box rest and/or consumes large volumes of concentrated carbohydrate feed, or the horse has dental disease and is unable to masticate properly. This condition could be diagnosed on rectal examination by a veterinarian. Impactions are often associated with the winter months because horses do not drink as much water and eat drier material (hay instead of grass), producing drier intestinal contents that are more likely to cause a blockage.

The primary pathophysiological abnormality caused by this obstruction is related to the trapping of fluid within the intestine oral to the obstruction. This is due to the large amount of fluid produced in the upper gastrointestinal tract, and the fact that this is primarily re-absorbed in parts of the intestine downstream from the obstruction. The first problem with this degree of fluid loss from circulation is one of decreased plasma volume, leading to a reduced cardiac output, and acid-base disturbances.

The intestine becomes distended due to the trapped fluid and gas production from bacteria. It is this distension, and subsequent activation of stretch receptors within the intestinal wall, that leads to the associated pain. With progressive distension of the intestinal wall, there is occlusion of blood vessels, firstly the less rigid veins, then arteries. This impairment of blood supply leads to hyperemia and congestion, and ultimately to ischaemic necrosis and cellular death. The poor blood supply also has effects on the vascular endothelium, leading to an increased permeability which first leaks plasma and eventually blood into the intestinal lumen. In the opposite fashion, gram-negative bacteria and endotoxins can enter the bloodstream, leading to further systemic effects.

### Strangulating obstruction.

Strangulating obstructions have all the same pathological features as a simple obstruction, but the blood supply is immediately affected. Both arteries and veins may be affected immediately, or progressively as in simple obstruction. Common causes of strangulating obstruction are intussusceptions, torsion or volvulus, and displacement of intestine through a hole, such as a hernia, a mesenteric rent, or the epiploic foramen.

### Non-strangulating infarction.

In a non-strangulating infarction, blood supply to a section of intestine is occluded, without any obstruction to ingesta present within the intestinal lumen. The most common cause is infection with Strongylus vulgaris larvae, which primarily develop within the cranial mesenteric artery.

### Inflammation or ulceration of the gastrointestinal tract.

Inflammation along any portion of the gastrointestinal tract can lead to colic. This leads to pain and possibly stasis of peristalsis (Ileus), which can cause excessive accumulation of fluid in the gastrointestinal tract. This is a functional rather than mechanical blockage of the intestine, but like the mechanical blockage seen with simple obstructions, it can have serious effects including severe dehydration. Inflammation of the bowel may lead to increased permeability and subsequent endotoxemia. The underlying cause of inflammation may be due to infection, toxin, or trauma, and may require special treatment in order to resolve the colic.

Ulceration of the mucosal surface occurs very commonly in the stomach (gastric ulceration), due to damage from hydrochloric acid produced in the stomach or alteration in protective mechanisms of the stomach, and is usually not life-threatening. The right dorsal colon may also develop ulceration, usually secondary to excessive NSAID use, which alters the homeostatic balance of prostaglandins that protect the mucosa.

### The Treatment of Colic

Colic may be managed medically or surgically. Severe clinical signs often suggest the need for surgery, especially if they cannot be controlled with analgesics. Immediate surgical intervention may be required, but surgery can be counter-indicated in some cases of colic, so diagnostic tests are used to help discover the cause of the colic and guide the practitioner in determining the need for surgery. The majority of colics (approximately 90%) can be successfully managed medically.

### Analgesia and sedation

The intensity of medical management is dependent on the severity of the colic, its cause, and the financial capabilities of the owner. At the most basic level, analgesia and sedation is administered to the horse. The most commonly used analgesics for colic pain in horses are NSAIDs, such as flunixin meglumine, although opioids such as butorphanol may be used if the pain is more severe. Butrophanol is often given with alpha-2 agonists such as xylazine and detomidine to prolong the analgesic effects of the opioid. Early colic signs may be masked with the use of NSAIDs, so some practitioners prefer to examine the horse before they are given by the owner.

### Fluid support

Fluids are commonly given, either orally by nasogastric tube or by intravenous catheter, to restore proper hydration and electrolyte balance. In cases of strangulating obstruction or enteritis, the intestine will have decreased absorption and increased secretion of fluid into the intestinal lumen, making oral fluids ineffective and possibly dangerous if they cause gastric distention and rupture. This process of secretion into the intestinal lumen leads to dehydration, and these horse require large amounts of IV fluids to prevent hypotension and subsequent cardiovascular collapse. Fluid rates are calculated by adding the fluid lost during each collection of gastric reflux to the daily maintenance requirement of the horse. Due to the fact that horses absorb water in the cecum and colon, the IV fluid requirement of horses with simple obstruction is dependent on the location of the obstruction. Those that are obstructed further distally, such as at the pelvic flexure, are able to absorb more oral fluid than those obstructed in the small intestine, and therefore require less IV fluid support. Impactions are usually managed with fluids for 3-5 days before surgery is considered. Fluids are given based on results of the physical examination, such as mucous membrane quality, PCV, and electrolyte levels. Horses in circulatory shock, such as those suffering from edotoxemia, require very high rates of IV fluid administration. Oral fluids via nasogastric tube are often given in the case of impactions to help lubricate the obstruction. Oral fluids should not be given if significant amounts of nasogastric reflux are obtained. Access to food and water will often be denied to allow careful monitoring and administration of what is taken in by the horse.

### Intestinal lubricants and laxatives

In additional to fluid support, impactions are often treated with intestinal lubricants and laxatives to help move the obstruction along. Mineral oil is the most commonly used lubricant for large colon impactions, and is administered via nasogastric tube, up to 4 litres once or twice daily. It helps coat the intestine, but is not very effective for severe impactions or sand colic since it may simply bypass the obstruction. Mineral oil has the added benefit of crudely measuring gastrointestinal transit time, a process which normally takes around 18 hours, since it is obvious when it is passed. The detergent dioctyl sodium sulphosuccinate (DDS) is also commonly given in oral fluids. It is more effective in softening an impaction than mineral oil and helps stimulate intestinal motility, but can inhibit fluid absorption from the intestine and is potentially toxic so is only given in small amounts, two separate times 48 hours apart. Epsom salts are also useful for impactions, since they act both as an osmotic agent, to increase fluid in the Gl tract, and as a laxative, but do run the risk of dehydration and diarrhoea. Strong laxatives are not recommended for treating impactions.

Examples of the compositions suitable for horses are described in the following patents, patent applications and reference documents: US20100291050; US20090181114; US20080095881; WO2014035470; US6827965; AU2011211461; WO2008041835; Pieretti and Gai, Animal Feed Science and Techology 148 (2009) 267-275 and "Tri-omega supplement ED - Squires Edward" Journal of Equine Veterinary Science, 18 (1998) 367.

There has now been developed a horse food which overcomes or substantially mitigates the above-mentioned and/or other disadvantages associated with the prior art.

### Summary of the Invention

According to the invention there is provided an animal foodstuff wherein the animal is an equid, the animal food stuff being characterised in that it comprises;
4% to 25% (w:w) milled chia seed;
45% to 95% (w:w) of alfalfa, and
2% to 20% (w:w) of oats.

The invention is advantageous because the milled chia seed forms a gel on contact with the water. This gel maintains the alfalfa and the oats in aqueous suspension. When drunk, the aqueous suspension passes through the animal's digestive system. The gel has inherent water holding capacity and an ability to hold within its molecular structure the alfalfa component and the oats component. As a result the aqueous suspension is carried predominantly intact through the small intestine and into the hind gut of the animal after ingestion. Whilst the small intestine can provide water to the animal immediately it has no water storage capacity and any excess is excreted through the renal system. This is compared to the hind gut which acts as a reservoir that can be repeatedly called on to replenish the interstitial fluid. As the majority of the aqueous suspension is delivered to this area it increases the hind gut fluid pool. The foodstuff therefore provides an effective dietary regimen for maintaining hydration of an animal and reducing the effects of dehydration whilst also simultaneously administering complete nutrition to the animal.

The term animal in this specification refers to horses.

Alfalfa which is low in carbohydrate levels, provides the animal with high fibre slow release energy from hindgut fermentation reducing the need for insulin producing carbohydrates within a food ration. Preferably the animal foodstuff comprises from 75% to 95% (w:w) of the alfalfa component. More preferably the animal foodstuff comprises from 80% to 90% (w:w) of the alfalfa component. This provides the correct amount of fibre to the animal's food. The alfalfa component may be milled, crushed or ground. Straw includes the dry stalks of cereal plants, after the grain and chaff have been removed.

The oat component preferably provides a carbohydrate source to the foodstuff, but may also provide a protein source. Typical protein levels of wheat, corn, oats and barley are 14%, 14%, 15.3%, and 9.2% respectively. The animal foodstuff comprises from 2% to 20% (w:w) of the oat component. More preferably the animal foodstuff comprises from 5% to 15% (w:w) of the oat component. This provides the correct amount of carbohydrate to the animal's food. The oat component may be milled, crushed or ground.

The animal foodstuff comprises 4% to 25%, more preferably 20%, of the milled chia seed component. Preferably the milled chia seed component is in the form of a powder. This maximises the surface area so that the rate of gel forming is increased, and the amount of water taken up to form the gel is increased. The edible milled chia seed component, when formed into a gel and ingested by the animal as the foodstuff also forms a physical barrier between carbohydrates and the digestive enzymes that break them down. This slows the conversion of carbohydrates into sugar, a more even blood-sugar level is achieved. This is of great benefit to insulin-resistant animals whose lives depend on low carbohydrate nutrition.

Any combination of ingredients may be added as long as they provide the resultant properties described and within the scope of the claims.

The Chia seed that produces the mucilage contains a fibre-rich fraction (FRF) of 29.56g/100g crude fibre content and 56.46g/100g total dietary fibre (TDF) content, of which 53.45g/100g is insoluble dietary fibre (IDF) and 3.01g/100g is soluble dietary fibre (SDF). By diluting more readily fermentable materials, fibre suppresses a rapid fall in pH of the large intestinal contents acting as a buffer to acid production and increases intestinal transit time, delays gastric emptying and slows the rate of glucose absorption, thereby reducing cholesterol absorption. The carbohydrate-sugar rate conversion is slowed down by the increased amount of insoluble fibre allowing the avoidance of high blood sugar levels which can reduce the occurrence of insulin resistance. The foodstuff of the present invention comprises both soluble and insoluble fibre provided by the addition of alfalfa meal (Medicago sativa) and by the polysaccharide mucilage formed by the Chia seed capsule.

Preferably the animal foodstuff forms a semi liquid drink on contact with water. That is to say that the aqueous suspension is of sufficient consistency so that it may be taken up by drinking by an animal. Preferably the foodstuff is prepared by mixing together the alfalfa component, the oat component and the chia component to form a base material. The base material is then preferably mixed with water in a ratio of between 9 and 21 parts water to 1 part base material. In this ratio, a sufficient amount of chia component is present to maintain the alfalfa component and the oat component in aqueous suspension. More preferably the base material is mixed with water in a ratio of 10 parts water to 1 part base material. Being a semi liquid drink, the foodstuff may be used in animals with compromised dentition. The molars found further back in the mouth affect the digestive capability of the animal. They are essential in the digestive process because they are used to grind feed into small particles. Inability to properly chew feeds might cause inefficient digestion in the gastrointestinal tract. The lifespan of these teeth (in horses for example) is usually 20 - 25 years. By the time the horse reaches this age, there is a real possibility of some molars loosening and falling out or teeth that cause pain reducing the desire to chew. Aged horses have a reduced digestive function in the large intestine. The foodstuff of the invention thus enables the horses to enjoy a palatable, readily digestible feed without the need to masticate, and reducing the probability of impaction colic.

An un-milled fibre component may be added to the foodstuff. This provides something for the animal to chew on when taking up the foodstuff and helps to control the intake of the foodstuff into the animal. This is particularly important in animals where lengthy chewing is required in maintaining a normal psychological state.

The animal foodstuff may be used in the hydration of animals. The animal foodstuff may increase the volume of the gastrointestinal fluid pool. One of the signs of dehydration in animals in increased heart rate. In exercising animals this is a particular problem. The inventors have surprisingly found that by feeding the foodstuff according to the invention to animals during and after vigorous exercise the animal's heart rate is reduced. The animal foodstuff may therefore reduce the heart rate of exercising animals.

The food stuff according to the invention may be advantageously used in horses with Equine Gastric Ulcers. Equines can develop gastric ulcers for many reasons and although mainly in the stomach and small intestine, they can be developed anywhere throughout the gastrointestinal tract. They are not specific and are found in foals as well as adult horses. To keep the gastrointestinal tract healthy horses should ideally have access to grazing around the clock as they produce hydrochloric acid (HCI) continuously. However this is not always possible due to the restrictions placed upon the domesticated animal.

If horses are allowed to spend long periods of time without food this continuous flow of acid can create lesions on the unprotected squamous epithelial tissue in the upper non glandular region of the stomach and the small intestine. This acid is used to break down carbohydrates and starches to be metabolised into glucose, however this acid can irritate these lesions causing excruciating pain. This is particularly true when large amounts of carbohydrates are fed, large amounts of HCI must be produced to break it down which again irritates these painful lesions.

Although the HCI acid produced can cause gastric ulcers the acidic environment is necessary to help protect the horse from proliferation of pathogenic bacteria such as E.Coli and Clostridium Difficile, prolonged use of gastric ulcer medications such as Omeprazole can also make these horses susceptible to illness if the delicate balance of pH is shifted to allow the proliferation of these pathogenic bacteria.

Another problem associated with using the gastric ulcer medications such as Omeprazole or any other proton pump inhibitors is that they inhibit the absorption of vitamin B12 and calcium. Studies in humans have shown that long term use of these proton pump inhibitors such as Omeprazole carries a 20% higher risk of bone fracture despite calcium supplementation.

The formulation of the soluble fibre gel attached to molecules of water suspends the fibre which provides a natural barrier to the HCI produced in the stomach for the breakdown of carbohydrates. Because of the consistency, the free flowing bolus lines the stomach and trickles through the small intestine simulating grazing and allows a more natural environment without interfering with the delicate balance of pH within the stomach allowing control of pathogenic bacteria and allowing for natural absorption of essential vitamins and minerals.

Since there is a lower level of carbohydrate in this formulation, less acid is produced combined with the lining effect of the gel, the inventor has found that the irritation of the lesions by the acid is reduced and surprisingly it has been shown to reduce the stereotypies associated with pain.

The foodstuff according to the invention is also advantageous because it can be used in horses with metabolic disorders. Equine Cushing's disease is a chronic progressive disease of the intermediate pituitary gland of older horses; Hyperglycemia and hyperinsulinemia are common findings in horses with Equine Cushing's. Providing calories from sources that do not contribute substantial quantities of glucose to the blood stream (e.g. fibre) appears to be necessary. The foodstuff according to the invention is very low in non-structural carbohydrate (NSC). The addition of low carbohydrate Alfalfa (Medicago sativa) meal to the formulation of the foodstuff provides the horse with high fibre slow release energy from hindgut fermentation reducing the need for insulin producing carbohydrates within a food ration.

The foodstuff according to the invention is also advantageous because it can be used in horses that are pregnant and/or lactating. During the first 8 months of pregnancy, mares should be fed the same as a normal idle or working mare of the same bodyweight of a non-pregnant mare. Access to ample amounts of good quality forage will generally provide sufficient dietary energy and protein as well as other nutrients to meet the mare's needs during the last 3 months of pregnancy and throughout lactation. However during the last trimester of pregnancy and lactation dietary energy requirements increase and therefore if this type of forage is not available then the mare may lose body weight and condition and both reproductive efficiency and milk production may be reduced. During the last 3 months of pregnancy and lactation the mare's requirement for digestible energy, protein, calcium and phosphorus increases substantially and therefore it may be necessary to supplement the mare's diet. The foodstuff of the invention provides 16.5% CP, 14.4% Oil, 0.77% calcium and 0.48% phosphorus, which provide a high energy dense, fibre based nutrition and a means of hydration on demand delivered in the form of a easily digested semi liquid drink.

The foodstuff according to the invention is also advantageous because it can be used in foals and youngstock. The management of foals is dependent on the age of the foal. With very young foals the dam's milk will normally provide all its nutritional need for the first 6-8 weeks of life. However the foal will begin to consume small portions of solid food shortly after birth. They will increasingly seek out solid feed to supplement their nutritional needs by consuming the dam's feed and it has been shown that foals that have become accustom to consuming hard feed prior to weaning show reduced weaning stress. Depending on the desired growth rate and post-weaning nutritional programme, traditionally foals have been fed on high energy dense creep feeds to supplement their diets providing 16 -20% high quality crude protein (CP), 0.8 - 1% calcium and 0.6 - 0.8 phosphorus. The foodstuff of the present invention provides 16.5% CP, 14.4% Oil, 0.77% calcium and 0.48% phosphorus, which provide a high energy dense, fibre based nutrition and a means of hydration on demand delivered in the form of a easily digested semi liquid drink.

The foodstuff according to the invention is also advantageously used in the management of colic. All cases of suspected colic should be referred to a veterinarian in the first instance, where diagnoses can be made and correct treatment can be determined. However, surprisingly, the inventor found that the properties of the foodstuff mixture provides a means of delivering hydration on demand in the very initial stages of colic if it is suspected that the symptoms suggest an impaction. Due to its highly palatable nature, it encourages horses to take in water in an attempt to soften the impaction by providing lubrication and fluid support or in the latter stages this fluid support can be administered via nasogastric intubation.

Because of the small particle size (< 2mm) of the alfalfa component, the chia component and the water component formed a suspension which does not separate into its individual components and is able to be passed down the nasogastric tube directly into the stomach which can provide hydration and can also deliver medication directly.

This method of hydrating is particularly important as a means of providing fluid support in the case of simple impactions by delivering fluid and electrolytes within the fibre component which also provides the roughage to support the motility of the gastrointestinal tract allowing peristalsis to assist the passing of the blockage.

A preferred embodiment of the invention will now be described in greater detail, by way of example only, with reference to the figures.

### Description of the Drawings

Figure 1 shows a graph of the absorption of water from the foodstuff on to an absorbent towel compared with the absorption of water from the component parts of the foodstuff.
Figure 2 shows the absorption rate of the individual components and that of the foodstuff after 10 hours.

### Detailed Description of an Embodiment of the Invention

### Example of Animal Food

To make up 1 kg of the foodstuff, 850g of milled alfalfa (A Poucher & Son), 100g of milled oats (Hamlins, Scotland) and 50g of powdered chia seed (The Chia Company) is taken. The ingredients are then mixed and screened to <2mm. 10 litres of water is then mixed into the raw material. After ten minutes the chia absorbs a considerable portion of the water and forms a mucilaginous gel. The gel maintains the alfalfa, and milled oats in aqueous solution which is in the form of a semi liquid drink. It is noted that no settling is experienced. Without the chia component the materials settle within the mixing container. The foodstuff mixture is then offered to a horse who proceeds to drink the suspension using a sucking action. In so doing the foodstuff is ingested.

The example above provides a source of plant based Omega 3 PUFA, with high levels of dietary fibre, good quality complete proteins and naturally occurring antioxidants such as phenolic glycoside-Q and K, chlorogenic acid, caffeic acid, quercetin and kaempferol.

The mucilage formed within the foodstuff of the invention is composed of polymers, most of them containing uronic acid units (mono carboxylic acids formerly derived by oxidation to a carboxylic of the terminal-CH2OH group of aldoses). They have the capacity to retain large amounts of water forming a very viscous and gelatinous gel.

The fibre content of the chia seed corresponds to a polysaccharide with a high molecular weight (0.8-2 x106 Daltons). The tentative structure of the basic unit of the polysaccharide is a tetra saccharide with 4-O-metil-ci-D-glucoronopyranosyl residues occurring as branches of 3-D-xylopyranosyl on the main chain. The mucilage has a chemical structure formed by heterogeneous polysaccharides and simple monosaccharide's corresponding to pentoses and hexoses; xylose is found in pentoses while galactose, rhamnose, mannose and glucose are found in hexoses.

The mucilage reaches maximum absorption level in 2 hours. After 7 hours the absorption becomes constant reaching 0.96g of water mass. On average Chia seed mucilage absorbs 0.62g of water mass, hydrating up to approximately 9.6 times its weight in water. The seeds are resistant to heat, light as well as temperature cycles and therefore no requirements or special storage conditions is needed.

Within the foodstuff mixture, the mucilage gel suspends the particles of the remaining components. The viscous gel suspends all the nutrients allowing all particles to be carried within it releasing water gradually as the bolus travels through the gastrointestinal tract.

The foodstuff mixture contains 32% polyunsaturated fat, of which 62% is omega-3. The high levels of oil provide high energy dense nutrition as well as the advantage of anti-inflammatory properties. The highly unsaturated omega-3 oils, contain natural antioxidants to protect against lipid oxidation, which causes oils to go rancid.

The foodstuff mixture contains 16.5% protein (but this may vary due to the growing conditions of the individual components). Unlike most other cereals the content of the foodstuff mixture is not limiting in any amino acids necessary in the diet which allows them to be utilized completely. Although the protein contained in whole Chia seeds has a relatively low digestibility of 29.01% when the seeds were ground into a powder, the digestibility increases to 79.80%. This increase in the digestible protein is due to the exposure of the seed contents allowing increased enzymatic action

The foodstuff mixture also contains niacin, riboflavin, and thiamin and the presence of macrominerals with calcium, potassium, and phosphorus being the most prominent.

**Table 1 Amino Acid Composition of Chia Seeds within the subject invention**

| **Amino Acid** | **% of Total Protein** | **Amino Acid** | **% of Total Protein** |
|---|---|---|---|
| Aspartic Acid | 9.47 | Isoleucine | 3.98 |
| Threonine | 4.25 | Leucine | 7.30 |
| Serine | 6.02 | Tyrosine | 3.41 |
| Glutamic Acid | 15.37 | Phenylalanine | 5.86 |
| Glycine | 5.23 | Lysine | 5.50 |
| Alanine | 5.34 | Histidine | 3.19 |
| Valine | 6.32 | Argenine | 11.03 |
| Cystine | 1.82 | Proline | 5.45 |
| Methionine | 0.45 | | |

The foodstuff mixture also has antioxidant properties. Antioxidants are a group of compounds that block the damaging effects of the so-called free radicals. The foodstuff mixture contains caffeic acid which is a dietary source of chlorogenic acid which removes all oxidative species and functions as an antioxidant reduces blood sugar levels.

The foodstuff mixture also comprises a number of vitamins. Vitamins are nutrients that are essential in the diet of horses; however the actual need for each differs considerably for normal bodily function. These requirements will usually be met by vitamins naturally present in a feedstuff, tissue synthesis, microbial synthesis and ultraviolet light. Dietary requirements for specific vitamins are affected by circumstance, for instance when a horse is kept indoors or has a very thick coat, the requirement for vitamin D will be greater than a horse that is field kept and exposed to sunlight. A foals dietary needs for B vitamins and vitamin K are higher due to their fast growth rate. Fat soluble vitamins include Vitamins A, D, E and K that are easily absorbed from the intestinal tract and stored within adipose tissue. The foodstuff mixture also provides all vitamins required by horses, however depending on the individual circumstances supplementation may be necessary as the levels of nutrients vary due to the growing conditions of the individual components.

The foodstuff mixture also provides the correct mineral balance to the animal. Although only a minor part of the diet, minerals play a crucial role in the health of animals such as horses. They are involved in numerous functions within the body such as acid base balance, formation of structural components, enzymatic functions and energy transfer. The mineral content of feeds and their bio-availability vary with soil mineral concentrations, plant species, stage of maturity and conditions at harvest. Minerals are elements that cannot be created or destroyed under normal circumstances and therefore must be provided in the diet. Minerals are typically classified as macrominerals, those typically needed in concentrations measured in g/kg or percentage, such as calcium, phosphorus, magnesium, potassium, sodium, chlorine and sulphur and microminerals, those measured in ppm or mg/kg such as cobalt, copper, iodine, iron, manganese, selenium, and zinc. There are other minerals of interest such as chromium, fluorine and silicon. The presence or lack of these minerals in the diet can cause clinical signs of excess or deficiency which can have a detrimental effect on the horse. The levels of minerals within the foodstuff are dependent on the bio-availability of minerals from the ingredients which are well documented.

**Table 2 Typical Proximate Analysis of the foodstuff of the invention**

| **Ingredient: Alfalfa meal 80%, Chia meal 10%, Oatmeal 10%** | |
|---|---|
| **Nutrient** | **%** |
| Moisture | 8.8 |
| Oil | 14.4 |
| Protein | 16.5 |
| Ash | 4.6 |
| Fibre | 18.8 |
| Calcium | 0.77 |
| Phosphorus | 0.48 |
| Sodium | 0.03 |
| Potassium | 0.79 |

### Example of animal food

In another example of the animal foodstuff according to the invention to make up 1 kg of raw material, 850g of milled alfalfa (A Poucher & Son), 100g of oat straw and 50g of powdered chia seed (The Chia Company) is taken. The ingredients are then mixed and screened to <2mm. 20 litres of water is then mixed into the raw material. After ten minutes the chia absorbs a considerable portion of the water and forms a mucilaginous gel. However, as compared to the example provided above this formulation is lower in the available carbohydrates such as sugars, starch or frutan. Thus this example of the animal foodstuff is particularly suited for feeding to animals who are suffering from laminitis. The animal foodstuff may therefore be used in the treatment of laminitis.

### Effect of foodstuff on Horses

The following is a series of letters sent to the inventor from individuals who have trialled the foodstuff according to the invention on their horses. It is noted that EquidGel is the trade name for the foodstuff according to the invention.

### Letter 1. Use in Endurance event horses.

"I am an Endurance GB rider and have been on the Scottish Endurance GB Team in 2013 for the home international event. I compete at Advanced level and most of my rides are between 65km and 120km in one day or 80km to 160km over 2 days. I started using EquidGel (trade name for the foodstuff according to the invention) at the start of the 2014 season. My horse is a 14.2 cob type weighing 475kg, and we struggle to keep him hydrated on long events ie 80km plus, as he retains the heat in his muscles. By feeding EquidGel this season I have noticed a big difference his final heart rates. His average heart rate after performing such an endurance race was 46 bpm which was 2 or 3 beats lower than when doing the same rides in the 2013 season (where he finished on rates of 48/49 bpm) when not being fed the foodstuff of the invention. This is why I am convinced the added hydration provided by EquidGel before, during and after my races has helped to lower his heart rate especially at the vet gate when a lower heart rate is critical. This season the weather was a lot warmer so I would have expected higher heart rates not lower, therefore I will continue to use this product and also to introduce it to my other endurance horse that is starting his competitive career next season."

### Letter 2. Use in a horse with a fractured jaw.

"I just had to write to say thank you for your product EquidGel. I don't often take pen to paper but I just had to write to you and compliment you on it. Please find below a statement regarding EquidGel: My horse is a Bay TB Mare 22yrs old 15.2hh 500kg and I have used EquidGel since May 2014 till present. The type of issue I had to face was a fractured jaw. Without question of doubt EquidGel has been extremely effective. So much so that my vet has now interested in the product for their equine hospital. The changes that I have witnessed are as follows - due to her injury my mare lost weight rapidly as she was unable to eat hay or haylage and struggled with hard feed, but she has now put the weight back in and is looking amazing thanks to EquidGel. Since I starting using the product I have now started feeding my other horse on it and have just placed my second order of 20kg and for 20kg of Equidchop too. My other GBSH gelding 17.2hh 12yr old 625kg BE Novice eventer is now also on EquidGel especially whilst eventing, as it is so easy to fed at an event to rehydrate him. If I had not had fed Equidgel I am sure that my mare would not have been in the position to be back under saddle.

### Letter 3. Use in a horse with eating disorders.

"I am a leisure rider. I have been feeding EquidGel for around 8 months. In fact I was one of the first to trial this revolutionary product. I used EquidGel for my 27 years old, 15.1hh cob and also on my friends 16.2hh ex racehorse, who is now 17 years old. He has always shown terrible cribbing and windsucking behaviour *[due to gastric ulcers]* and has had to wear a crib collar constantly. Since we removed all sweet feed and treats from his diet and put him on a complete EquidGel fibre diet, the difference has been remarkable, his windsucking has almost ceased and he very rarely has to wear his collar and his body condition has also improved considerably. My old cob looks so absolutely fabulous with a beautiful coat and is very calm and relaxed. Because both my cob and the TB do very little work, they do not need a great amount of feed so I buy 20kg every 2 months and the owner of the TB buys around the same amount. This will increase in the winter. This is the only feed we use and it saves me about £6 per month. I will continue to use EquidGel as I am keen to feed a completely natural feed for the health of my horses. I think EquidGel is the most important new horse feed on the market and I will continue to use this amazing product.

### Letter 4. Use in a horse during endurance events

"Just wanted to say how impressed I am with your product EquidGel. We have been using it for two of my horses since July, after being recommended by a fellow Endurance rider. One of the horse's, Reggie, is not exactly an endurance model so his management has been more tricky for us, especially this season when he has been aimed at upgrading to advanced status. He is a middleweight 16.2 gelding 50% Arab but 50% Warmblood so heavier and denser muscled than his pure bred, half-brother. One of the issues we have had with him is drinking during his competitions, as you will appreciate hydration is a major factor in gaining a good result. Once we started using EquidGel, he has gone from strength to strength; aided undoubtedly by the fact that he takes his EquidGel bucket with enthusiasm at every opportunity! We have had improved results, his in competition recoveries have been better, and quicker and his post competition recoveries amazing! In fact, he often has appeared to have no loss of condition or tiredness! The fact that your product maintains his vital hydration means his appetite is good and he maintains his optimum condition so easily! Our season target of upgrading to advanced was fulfilled in style with his final qualification, an 80km (50 mile) GER completed at higher speed and quicker recoveries than any other result throughout the season....he didn't look in the slightest bit tired! I have been involved in the sport of Endurance riding for almost 30 years and am so pleased that you have developed this product which will help so much with the care and condition of our competition horses! I was cautious about changing their diets to be exclusively EquidGel fed horses but can honestly say that both my boys looks amazing and clearly love their food! and happily also compares very favourably with cost to the feeds I previously used. I'm already looking forward to our 2015 season, when we hope both boys will be tackling some new challenges fuelled by EquidGel!"

### Water retention assay.

This assay demonstrates the water retention properties of the foodstuff according to the invention compared to each of the individual components comprised within it.

### Methods

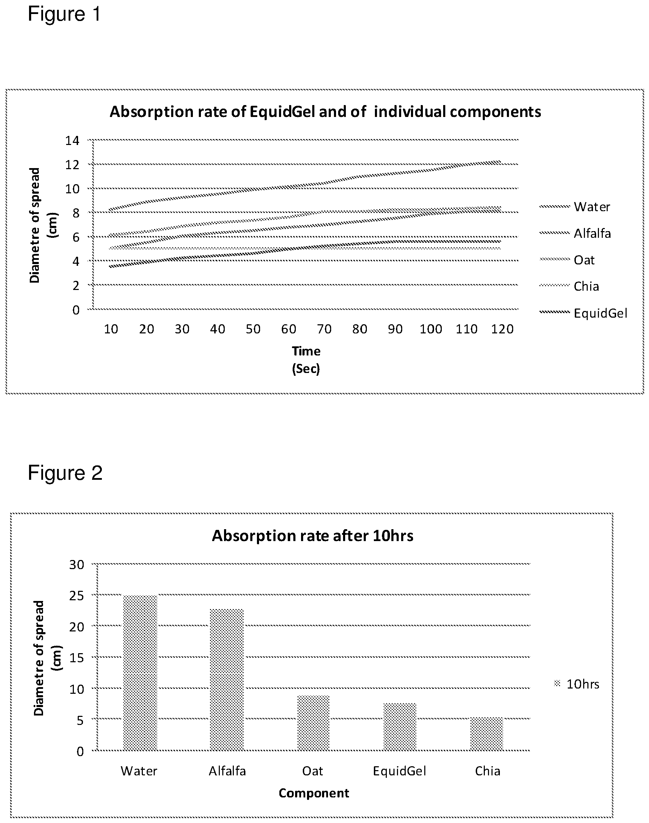

An assay was devised to show the rate at which water was released from the foodstuff after the foodstuff was placed on an absorbent material. In the study the absorbent material chosen was 10 sheets of two ply paper towel (overlaid), which was marked in 1 cm radial increments. The towel was used to simulate the semi permeable membrane of the gastrointestinal tract. In a control experiment, 25 ml of water was poured onto the middle of the towels and the absorption (indicated by the boundary of the wet material) was marked on the absorbent sheet at 10 second intervals for up to 2 minutes. The towels were left overnight for 10 hours. This experiment was carried out three times to get an average rate of absorption. The same methodology was repeated for each test subject of which included 2.5g Alfalfa chopped up to 5 mm lengths, 2.5g Finely Ground Oatmeal, 2.5g Finely Ground Chia or 2.5g Foodstuff of the invention (< 2mm screen). In each of the test cases each component was weighed and placed in a receptacle. One at a time, each component was added to a test tube containing 25ml of water and left for 5 minutes to allow each component to soak and to reach saturation. After 5 minutes each sample was poured onto the absorbent paper towel. As for the control study each test subject was repeated three times. The average results are shown in the table below. The results are also shown in Figure 1. The absorption after 10 hours in shown in Figure 2.

**Table 3. The diameter (cm) spread of water released from component.**

| | Time (seconds) | | | | | | | | | | | | |
|---|---|---|---|---|---|---|---|---|---|---|---|---|---|
| Material | 10 | 20 | 30 | 40 | 50 | 60 | 70 | 80 | 90 | 100 | 110 | 120 | 10hrs |
| Water | 8.2 | 8.8 | 9.2 | 9.5 | 9.8 | 10.1 | 10.4 | 10.9 | 11.2 | 11.5 | 11.9 | 12.2 | 25 |
| Alfalfa | 5 | 5.5 | 6 | 6.3 | 6.5 | 6.7 | 6.9 | 7.2 | 7.5 | 7.8 | 8 | 8.1 | 23 |
| Oat | 6.1 | 6.4 | 6.8 | 7.1 | 7.3 | 7.6 | 8 | 8 | 8.2 | 8.2 | 8.3 | 8.4 | 9 |
| Foodstuff of the invention | 3.5 | 3.8 | 4.2 | 4.4 | 4.6 | 4.9 | 5.2 | 5.4 | 5.6 | 5.6 | 5.6 | 5.6 | 7.7 |
| Chia | 5 | 5 | 5 | 5 | 5 | 5 | 5 | 5 | 5 | 5 | 5 | 5 | 5.5 |

The water spread up to 8cm in the first 10 seconds and continued to over 12cm within 2 minutes which shows a rate of 2cm/min. The water was left for 10 hours and when it spread to a diameter of 25cm. The water penetrated all ten layers of the absorbent paper. The water within the Alfalfa spread up to 6cm within the first 10 seconds and continued to 8.1cm which showed a rate of absorption of 1.55cm/min. The alfalfa was left for 10 hours on the absorbent paper and it felt dry to the touch. The spread diameter measured 23cm. The water also penetrated all ten layers of the absorbent paper. The water within the oat meal had a similar absorption rate as the alfalfa, starting at 5cm and finishing at 8.4 cm which gave a rate of absorption of 1.15cm/min. The oatmeal solution was left on the absorbent paper and felt dry to the touch but the oatmeal had absorbed more of the water. The spread diameter after 10 hours had continued to 9cm. The water had also penetrated all ten layers of the absorbent paper. The foodstuff of the invention, which is a combination of the individual components at a ratio of Alfalfa 85%, Oatmeal 10% and Chia 5%, spread up to 3.5 cm in the first 10 seconds and then continued to 5.6cm which gave an absorption rate of 1.05cm/min. The Foodstuff of the invention was left on the absorbent paper for 10 hours and felt moist to the touch and when squeezed water was still available. The spread diameter after 10 hours was 7.7cm. The water had also penetrated all ten layers of the absorbent paper. The Chia solution formed a viscous gel which when placed on the absorbent paper started at 5 cm, however there was no spread of water. All the water was attached to the soluble fibre in the chia formed by the mucilage, there was no free water molecules available. No spread was recorded over the 2 min period; therefore Ocm/min was recorded. The Chia was left on the absorbent paper for 10 hours and felt wet and sticky to the touch however no water residue was felt. There was 0.5cm spread recorded after 10 hours and the water penetrated four layers of the absorbent paper. Almost all of the water was held within the mucilage and very little absorption was noted.

### Discussion

The water behaved as would be expected with maximum absorption through the simulated semi permeable membrane. The initial spread was very fast and as the water was absorbed it slowed down until all the water was absorbed into the membrane. This demonstrates that in vivo the majority of the water would be absorbed and carried through to the interstitial fluid not reaching the hindgut. After soaking for 5 minutes the Alfalfa reached saturation quickly as there is little soluble fibre to hold onto the water, therefore allowing lots of free water molecules available to be absorbed. The alfalfa and water solution acted in the same manner as the water with an initial immediate spread to 5cm however after the initial spread the water was released slower than the water alone at a rate of 1.55cm/min. However by 10 hours all the water was absorbed. In vivo this would mean that that most of the free water would be absorbed before reaching the hindgut. The oatmeal and water solution showed an initial immediate spread of 6.1cm which showed that there was little saturation during the 5 minute soaking period, however it looked as though some of the oatmeal had dissolved into the water solution, and however there was still free water available so the water retention within the oatmeal was minimal. The spread rate of 1.15cm/min showed that the free water molecules were absorbed quite quickly. In vivo this would mean that the majority of the water would have been absorbed into the interstitial fluid before reaching the hindgut. However over time the oatmeal seemed to absorb some water making it swell. After 10 hours the diameter of the spread was 9cm, which means that some water was retained within the oatmeal. With the ground Chia the surface area had been increased causing more soluble fibre to be available, the moment that the water was added the polysaccharides xylose and arabinose produce the mucilage which attracted all the free water molecules. Therefore saturation did not occur as there is an abundance of soluble fibre. This has a negative effect as the hydrophilic properties will keep attracting fluid until saturation occurs, therefore having the effect of drawing water from its surroundings. This within the digestive tract can be very detrimental as it may well cause dehydration if this component is solely taken into the body under saturated (Dry or without adequate water). After the 5 minute soaking period the foodstuff of the invention reached saturation however there was still free water molecules available for absorption into the interstitial fluid which is essential for servicing thermoregulation, saliva production, mucous membrane and other bodily fluids. However, the spread rate of the foodstuff of the invention was surprisingly much slower at 1.05cm/min, which shows that more water was being retained within the formulation for a longer period of time. After leaving the foodstuff of the invention for a further 10 hours the diameter of the spread was surprisingly only 7.7cm.

This demonstrates that the rate of water released onto the towels by the foodstuff of the invention was less than what would be expected from the individual rates of water released by water, alfalfa with water, oats with water and chia with water added together. It also demonstrates that the amount of water released onto the towels was by the foodstuff of the invention was less than what would be expected from the individual amounts of water released by water alone, alfalfa with water, oats with water and chia with water, added together. This represents a synergistic effect of the combination of all the components of the foodstuff.

Ultimately, the faster the rate of absorption in the gut the more water moves through and the shorter distance travelled, the slower the rate of absorption the less water moves through the membrane and the further into the digestive tract it its transported. The ratio of the components that make up the foodstuff of the invention allows adequate free water molecules to move through the semi permeable membrane into the interstitial fluid and due to the slower rate of absorption the formulation shows that the retention of fluid bound to soluble fibre would be carried further into the digestive system (to the hind gut) allowing it be retained much longer until metabolic breakdown releases the water molecules.

## Claims

1. An animal foodstuff wherein the animal is an equid, the animal food stuff being **characterised in that** it comprises;
4% to 25% (w:w) milled chia seed;
45% to 95% (w:w) of alfalfa, and
2% to 20% (w:w) of oats.

2. An animal foodstuff according to any preceding claim, wherein the animal foodstuff forms a semi liquid drink on contact with water.

3. An animal foodstuff according to any preceding claim wherein the foodstuff is prepared by mixing together the alfalfa, the oats and the chia seed to form a base material, the base material then being mixed with water in a ratio of between 9 and 21 parts water to 1 part base material.

4. An animal foodstuff according to any preceding claim, wherein the oats is in the form of oat straw.

## Patentansprüche

1. Tierfuttermittel für Einhufer, wobei das Tierfuttermittel **dadurch gekennzeichnet ist, dass** dieses umfasst:
4% bis 25% (Gew.:Gew.) gemahlene Chiasamen;
45% bis 95% (Gew.:Gew.) Alfalfa, und
2% bis 20% (Gew.:Gew.) Hafer.

2. Tierfutter nach dem vorgenannten Anspruch, wobei das Tierfuttermittel bei Kontakt mit Wasser ein halbflüssiges Getränk bildet.

3. Tierfuttermittel nach einem der vorhergehenden Ansprüche, wobei das Futtermittel zubereitet ist durch Mischen von Alfalfa, Hafer und den Chiasamen, um ein Basismaterial zu bilden, wobei das Basismaterial dann mit Wasser in einem Verhältnis zwischen 9 und 21 Teilen Wasser zu 1 Teil Basismaterial gemischt wird.

4. Tierfuttermittel nach einem der vorhergehenden Ansprüche, wobei der Hafer in Form von Haferstroh enthalten ist.

## Revendications

1. Aliment pour animaux, l' animal étant un équidé, l'aliment pour animaux étant **caractérisé en ce qu'**il comprend :
de 4 % à 25 % (en poids) de graines de chia moulues ;
de 45 % à 95 % (en poids) de luzerne ; et
de 2 % à 20 % (en poids) d'avoine.

2. Aliment pour animaux selon la revendication précédente, l'aliment pour animaux formant une boisson semi-liquide en contact avec l'eau.

3. Aliment pour animaux selon l'une quelconque des revendications précédentes, l'aliment étant préparé par mélange ensemble de la luzerne, de l'avoine et des graines de chia pour former une matière de base, la matière de base étant ensuite mélangée avec de l'eau à un rapport compris entre 9 et 21 parts d'eau pour 1 part de matière de base.

4. Aliment pour animaux selon l'une quelconque des revendications précédentes, dans lequel l'avoine est sous la forme de paille d'avoine.
